# EUROPEAN PATENT SPECIFICATION

(11) **EP 3 389 472 B1**
(45) Date of publication and mention of the grant of the patent: **08.05.2019**
(21) Application number: 16806093.7
(22) Date of filing: 06.12.2016
(51) Int. Cl.: A61B 5/00, A61B 5/1455, A61B 5/145, A61B 5/16

(54) **WEARABLE DEVICE AND METHOD FOR DETERMINING ELECTRO-DERMAL ACTIVITY OF A SUBJECT**
TRAGBARE VORRICHTUNG UND VERFAHREN ZUR BESTIMMUNG DER ELEKTRODERMALEN AKTIVITÄT EINES SUBJEKTS
DISPOSITIF PORTABLE ET PROCÉDÉ DE DÉTERMINATION DE L'ACTIVITÉ ÉLECTRO-DERMALE D'UN SUJET

(30) Priority: 14.12.2015 EP 15199717
(43) Date of publication of application: 24.10.2018
(73) Proprietor: Koninklijke Philips N.V., 5656 AE Eindhoven (NL)
(72) Inventor: AARTS, Ronaldus Maria, 5656 AE Eindhoven (NL); PRESURA, Cristian Nicolae, 5656 AE Eindhoven (NL); NICOLAE, Andrei, 5656 AE Eindhoven (NL)
(74) Representative: Coops, Peter
(86) International application number: PCT/EP2016/079802
(87) International publication number: WO 2017/102412

(56) References cited:
- JP-A- S61 198 039
- US-A1- 2003 139 654
- US-A1- 2009 269 003
- US-A1- 2009 318 908
- US-A1- 2012 041 283
- US-A1- 2014 155 724

## Description

### FIELD OF THE INVENTION

The present invention relates to a wearable device and a corresponding method for determining electro-dermal activity of a subject.

### BACKGROUND OF THE INVENTION

In general, regulation of physiological states of arousal is achieved by a balance of activity within sympathetic and parasympathetic subdivisions of the autonomic nervous system (ANS). While the parasympathetic nervous system promotes restoration and conservation of bodily energy, the sympathetic nervous system stimulates increased metabolic output to deal with external challenges. As such, increased sympathetic activity (sympathetic arousal) elevates heart rate, blood pressure, and sweating, as well as redirects blood from the intestinal reservoir toward skeletal muscles, lungs, heart, and brain in preparation for motor action. Sympathetic postganglionic fibers consisting of non-myelinated class C nerve fibers surround eccrine sweat glands and their activity modulates sweat secretion. Since sweat is a weak electrolyte and good conductor, the filling of sweat ducts results in many low-resistance parallel pathways, thereby increasing the conductance of an applied current. Changes in skin conductance at the surface, referred to as electro-dermal activity (EDA), reflect activity within the sympatheticaxis of the ANS and provide a sensitive and convenient measure of assessing alterations in sympathetic arousal associated with emotion, cognition, and attention.

Stress is generally defined as a disruption of the autonomic balance involving a state of high sympathetic activation. Since EDA is solely determined by the activity of the sympathetic branch of the ANS, which is predominant in stress states, tonic EDA parameters may be regarded as suitable measures of ANS activity induced by stress. Autonomic responses in the skin such as sweating, piloerection, and vasomotor changes can thus be elicited by various emotional states via the Papez circuit in the limbic system. In addition, it is widely recognized that attention-grabbing stimuli and attention-demanding tasks also evoke increased EDA responses. Despite improvements in measuring equipment since the discovery of electro-dermal phenomena more than 100 years ago, much of the research in this area is limited to observational measurements performed over short periods of time in laboratory settings or artificial clinical environments.

The need for monitoring patients over extensive periods of time has stimulated interest in wearable technologies - unobtrusive wearable devices that can be worn during normal daily activity to gather physiological data over periods of several weeks or months. Long-term monitoring of EDA will allow the observation of patterns of sympathetic arousal and regulation at a significantly longer time scale (days to months) compared to existing studies (minutes to hours) and could potentially reveal previously unobservable trends. In addition, long-term measurements taken in a person's natural home environment also provide a clearer picture of the person's physiological state than a short period of assessment in an unnatural clinical setting. Clinically, wearable EDA sensors can be used in psychopathology, dermatology, and neurology for diagnostic purposes and therapy evaluation.

WO 2007/144817 A1 discloses a skin monitoring device for application near the skin, comprising a processing circuit connecting means and at least one photosensor configured to detect at least one approximate wavelength of light reflected and/or emitted by the skin, wherein a signal receiving circuit is provided for receiving signals from the at least one photosensor.

### SUMMARY OF THE INVENTION

It is an object of the present invention to provide an improved wearable device and a corresponding method for determining electro-dermal activity of a subject with higher reliability and accuracy.

In a first aspect of the present invention there is presented a wearable device for determining electro-dermal activity of a subject, said wearable device comprising
- a light source for emitting light including infrared light in the wavelength range between 750 and 950 nm into tissue of the subject,
- a light sensor for receiving at least part of the emitted light after an interaction of the emitted light with the tissue,
- an evaluation unit for determining the electro-dermal activity from the received light, wherein said evaluation unit is configured to evaluate only infrared light in the wavelength range between 750 and 950 nm, and
- a support for carrying the light source, the light sensor and the evaluation unit, wherein the light source and the light sensor are arranged at a predetermined distance from each other.

In another aspect of the present invention there is presented a corresponding method for determining electro-dermal activity of a subject.

In contrast to most common ways for measuring the electro-dermal activity (EDA) by injecting an electric current into the skin, which is intrusive, the device and method according to the present invention the measurement uses an optical principle, particularly by use of infrared light. This principle is passive since the light emitted into the skin is harmless. This optical principle for monitoring vital signs, e.g. heart rate, SpO2, etc., of a subject is generally known and used in wearable devices, such as a wrist-worn device, a finger-clip device, etc. Hence, such a known wearable device may also be modified as disclosed herein to be able to determine a subject's EDA, in particular by making use of infrared light in a particular region of the spectrum. In other embodiment a wearable device may solely be constructed for this purpose, or other existing wearable devices may be provided with additional means as disclosed herein.

Other disadvantages overcome by the proposed solution include wearing off of the electrodes needed for measuring electrical activity, and the interference of the electrical signals with the hair, which is not the case for the optical sensor, since hair is mostly transparent for infrared light.

Sweat does not flow continuously through the duct to the skin surface, but rather in a pulsatile manner with pulsations of 12 - 21 Hz. Rhythmic contractions of the myoepithelia, surrounding not only the secretory but also the ductal part of the sweat gland like a helix, are regarded as the source of the pulse.

According to the present invention a particular part of the infrared spectrum is evaluated, in particular a wavelength range between 750 and 950 nm, which has been found to see the correlation between the sensed light and the subject's electro-dermal activity. This wavelength range has the further advantage that the heart beat pulse is less visible so that it would not interfere with the measurement signal, and further, that the measurement signal does not accumulate noise.

In some embodiments the light source, in particular a light source (e.g. an LED) for emitting incoherent light, the light sensor (e.g. a photodiode) and/or the evaluation unit (e.g. a processor) are configured to emit, receive and/or evaluate only infrared light in the wavelength range between 750 and 950 nm, in particular in the wavelength range between 775 and 825 nm or at a wavelength of 800 nm. In particular a wavelength range of 800 nm (± 50 nm) has been found to deliver the best results.

In another embodiment the light source and the light sensor are arranged at a distance (preferably between their centers) in the range between 0.5 mm and 5 mm, in particular in the range between 1 mm and 4 mm, from each other. It has been found that the distance has a large impact on the reliability and accuracy of the measurement. The optimal distance of the light source and the light sensor, which are preferably arranged such that they are in contact with the skin (or at least in close proximity (i.e. less than 2 mm) to the skin) when the wearable device is worn by the subject, has been found to be in the range of 4 mm. At a very small distance or the desired effect was too small or even not measurable. The same holds for much larger distances, where the light scattered or reflected by the skin and/or tissue would even not sufficiently reach the light sensor.

In another embodiment said evaluation unit is configured to evaluate the intensity of the received light to determine the electro-dermal activity. The amplitude of the measured signal (i.e. the received light) is correlated with the EDA and is thus preferably used to determine the subject's EDA. The amplitude of the received signals may generally depend on the color used, the time of the measurement and the properties of the physical layer that created EDA (like sweat). In principle, the spectroscopic results can be used to determine what type of sweat it is, leaving open the possibility to discriminate the physical circumstances creating the EDA (emotion, stress, sport, etc.). Generally, a trend of the EDA may be determined over time and a threshold may be used to decide if there is EDA or not.

The wearable device may further comprise a holding element for holding the wearable device at the subject's body. Options for holding elements may include a wristband, a belt, a sticker, a band, or a clip, but other options are possible. Further, the holding element may also be an external element provided separately and/or not fixedly arranged at the wearable device.

Still further, the wearable device may further comprise a user interface for issuing information about the determined electro-dermal activity. Such a user interface may e.g. include a display or loudspeaker, but may also include a transmitter for transmitting the output to an external device, such as a computer, laptop, smartphone, physician's workstation, etc. for being issued and/or processed there.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a wearable device according to present invention,
Fig. 2 shows a schematic diagram of a second embodiment of a wearable device according to present invention, and
Fig. 3 shows a flowchart of a method according to the present invention.

### DETAILED DESCRIPTION OF THE INVENTION

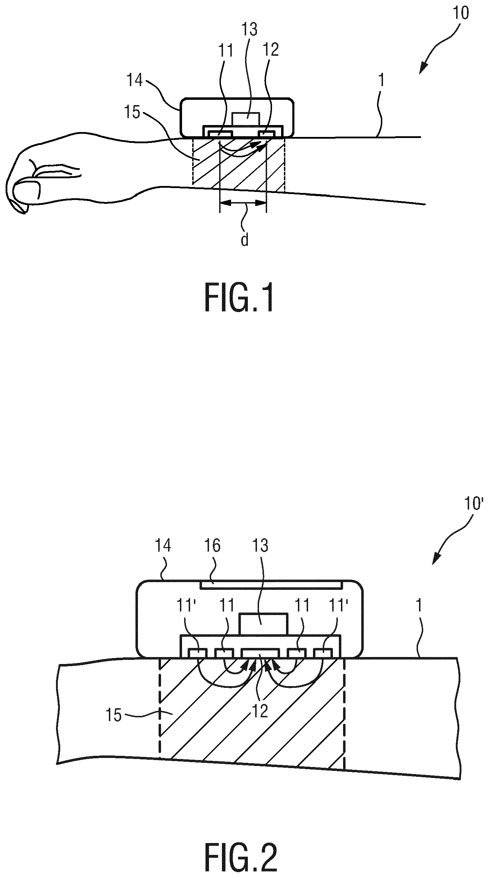

Fig. 1 shows a schematic diagram of a first embodiment of a wearable device 10 according to present invention for determining electro-dermal activity of a subject 1. The wearable device 10 comprises a light source 11 for emitting light including infrared light in the wavelength range between 750 and 950 nm into tissue of the subject 1, a light sensor 12 for receiving at least part of the emitted light after an interaction of the emitted light with the tissue, and an evaluation unit 13 for determining the electro-dermal activity from the received light. A support 14, such as a casing or housing, is provided for carrying the light source 11, the light sensor 12 and the evaluation unit 13, wherein the light source and the light sensor are arranged at a predetermined distance from each other.

The light source 11 may comprise one (or more) LED(s), e.g. infrared LED(s), that emit(s) light into a skin portion of the subject 1, e.g., the skin portion at the subject's wrist or face. This emitted light interacts with the tissue of the subject 1 and in particular with the pulsating blood in this tissue. Part of the emitted light may be transmitted through the tissue and part of it may be reflected. After this interaction, at least part of the emitted light is received by means of the light sensor 12, e.g. a photosensor.

Based upon the received light, information about the electro-dermal activity of the subject is derived in the evaluation unit 13, e.g. a processor. In particular, changes in the intensity of the light received by the light sensor 12 are correlated with the electro-dermal activity. E.g. a sudden increase in the received light signal, which lasted few tens of seconds, may be seen if the electro-dermal activity suddenly changes. This may particularly be a response from the sweat accumulated at the moment.

Usually, the light source 11 includes an LED, which may be operated in pulsed mode, i.e. emits light in short pulses. This may also be referred to as burst mode. The LED may be operated periodically, e.g. 0.5 ms on per 8 ms period time. Continuously operating the optical sensor arrangement particularly refers to operating the LED in such a pulsed mode. Normally, it is not required that the LED is powered on all the time. Power considerations require the LED to be in its on state as little as possible. It may, however, also be possible that the LED is operated in a continuous mode, i.e. always on.

An embodiment of the wearable device 10 is a wrist-worn device as shown in Fig. 1. The wearable device 10 is placed, preferably, above the radial artery of the wrist, because there the electro dermal response is maximal. Others preferred sites may be the flexor side of fingers and toes, and the forehead.

For holding the wearable device 10 at the respective subject's body part the wearable device 10 further comprises a holding element 15, which may e.g. comprise a wristband, a belt, a sticker, a band, or a clip.

The distance d between the light source and the light sensor (both preferably in contact with the skin or at a small distance from the skin less than 5 mm, preferably less than 1mm). The distance d is preferably the distance between the centers of the light source and the light sensor and is in the range between 0.5 mm and 5 mm, in particular in the range between 1 mm and 4 mm, from each other. An optimal distance d may e.g. be about 4mm. If the distance is smaller than 1 mm, the desired effect may not be present or not strong enough. If the distance is larger than 5mm, the light would be lost into the skin, and few light would reach the light sensor.

Fig. 2 shows a schematic diagram of a second embodiment of a wearable device 10' according to present invention. According to this embodiment the sweat (electro-dermal activity) is measured by means not only of a single wavelength (or wavelength range), in particular an infrared wavelength, but via at least a second wavelength (or wavelength range). For this purpose the wearable device 10' comprises at least a second light source 11' for emitting light at a second wavelength (or wavelength range), e.g. in a wavelength range of green light. In this way, the richer content of the sweat spectrum can be addressed, and additional properties of the sweat can be measured additionally, like the salt content.

The advantage of using a multicolor approach is robustness against different artifacts, like motion artifacts. In such cases, all wavelength channels of the wearable device will present the same artifact, and the wearable device will be configured to filter that data.

From each light source 11, 11', not only a single element may be provided, but two or more of the same type may be provided to increase the amount of light emitted into the tissue.

In another embodiment a single light source is provided for emitting light in a broad (full) spectrum, wherein the light sensor 12 is configured to sense only light in the desired part(s) of the spectrum and/or wherein the evaluation unit is configured to evaluate only sensed light in the desired part(s) of the spectrum.

Further, in the embodiment shown in Fig. 2 the wearable device 10' further comprises a user interface 16 for issuing information about the determined electro-dermal activity, e.g. a value indicative of the strength of the electro-dermal activity or an indicator indicating the trend (e.g. rising, falling, stable, etc.) of the strength of the electro-dermal activity. Such an interface may comprise a display. In other embodiments a transmission unit may be provided for transmitting the information to another entity, e.g. a smartphone, a workstation, a computer, etc. for issuance or further processing.

The wearable device itself can be placed on other parts of the skin (like forehead, or foot), which respond strongly with sweat to emotions. Further, the wearable device can be also integrated into garments like helmets (for athletes), headbands, gloves, jewelry, glasses (like google glasses), into the bra or in the sole (because the lower part of the feet respond also well with sweat to emotions).

The wearable device may be worn at the radial artery. Because the wrist-worn device representing the wearable device may change its position during wearing, some measures may be taken additionally to keep the same location of the measurement. For example, the wrist-worn device may be tightened. Further, a multisensory approach may be applied, where more sensors measure around radial artery and the best signal is chosen. This embodiment can be extended further with a camera in contact with the skin.

The present invention seeks to detect a fluid layer or galvanic skin response of the skin. It is the property of the human body that causes continuous variation in the electrical characteristics of the skin. Conventionally, electro-dermal activity has also been known as skin conductance, galvanic skin response (GSR), electro-dermal response (EDR), psycho-galvanic reflex (PGR), skin conductance response (SCR), and skin conductance level (SCL).

Incoherent light is emitted into the skin (preferably via one or more LEDs). The light is preferably chosen in a wavelength of the infrared light where the heart beat pulse was less visible. A preferred wavelength range is between 750 and 950 nm, in particular a wavelength range between 775 and 825 nm or at a wavelength of 800 nm. With this choice the light does not interfere with the measurement. The wavelength shall also not very because the signal would accumulate noise that would be less than 900nm. It turned out that 800 nm (plus/min 50nm) is a good choice.

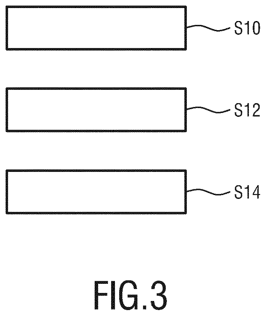

Fig. 3 shows a flowchart of a method according to the present invention for determining electro-dermal activity of a subject. In a first step S10 light including infrared light in the wavelength range between 750 and 950 nm is emitted into tissue of the subject. In a second step S12 at least part of the emitted light is received after an interaction of the emitted light with the tissue. In a third step S14 the electro-dermal activity is determined from the received light.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Wearable device for determining electro-dermal activity of a subject (1), said wearable device (10) comprising:
- a light source (11) for emitting light including infrared light in the wavelength range between 750 and 950 nm into tissue of the subject,
- a light sensor (12) for receiving at least part of the emitted light after an interaction of the emitted light with the tissue,
- an evaluation unit (13) for determining the electro-dermal activity from the received light, wherein said evaluation unit (12) is configured to evaluate only infrared light in the wavelength range between 750 and 950 nm, and
- a support (14) for carrying the light source (11), the light sensor (12) and the evaluation unit (13), wherein the light source and the light sensor are arranged at a predetermined distance from each other.

2. Wearable device as claimed in claim 1,
wherein said light source (11) is configured to emit only infrared light in the wavelength range between 750 and 950 nm, in particular in the wavelength range between 775 and 825 nm or at a wavelength of 800 nm.

3. Wearable device as claimed in claim 1,
wherein said light sensor (12) is configured to receive only infrared light in the wavelength range between 750 and 950 nm, in particular in the wavelength range between 775 and 825 nm or at a wavelength of 800 nm.

4. Wearable device as claimed in claim 1,
wherein said evaluation unit (12) is further configured to evaluate only infrared light in the wavelength range between 775 and 825 nm or at a wavelength of 800 nm.

5. Wearable device as claimed in claim 1,
wherein the light source (11) and the light sensor (12) are arranged at a distance in the range between 0.5 mm and 5 mm, in particular in the range between 1 mm and 4 mm, from each other.

6. Wearable device as claimed in claim 1,
wherein said evaluation unit (14) is configured to evaluate the intensity of the received light to determine the electro-dermal activity.

7. Wearable device as claimed in claim 1,
further comprising a holding element (15) for holding the wearable device at the subject's body.

8. Wearable device as claimed in claim 7,
wherein said holding element (15) comprises a wristband, a belt, a sticker, a band, or a clip.

9. Wearable device as claimed in claim 1,
further comprising a user interface (16) for issuing information about the determined electro-dermal activity.

10. Method for determining electro-dermal activity of a subject (1), said method comprising:
- emitting light, by a light source (11) including infrared light in the wavelength range between 750 and 950 nm into tissue of the subject,
- receiving, by a light sensor (12) at least part of the emitted light after an interaction of the emitted light with the tissue, and
- determining, by an evaluation unit (13), the electro-dermal activity from the received light, wherein the determination is based on evaluation of only infrared light in the wavelength range between 750 and 950 nm,
wherein the light source (11), the light sensor (12) and the evaluation unit (13) are carried by a support (14) and wherein the light source and the light sensor are arranged at a predetermined distance from each other.

11. The method according to claim 10 further comprising evaluating only infrared light in the wavelength range between 775 and 825 nm or at a wavelength of 800 nm.

12. Computer program comprising program code means for causing a device according to claim 1 to carry out the steps of the method as claimed in claims 10 or 11.

## Patentansprüche

1. Tragbare Vorrichtung zum Ermitteln einer elektrodermalen Aktivität eines Subjekts (1), die tragbare Vorrichtung (10) umfassend:
- eine Lichtquelle (11) zum Ausstrahlen von Licht, das infrarotes Licht im Wellenlängenbereich zwischen 750 und 950 nm enthält, in Gewebe des Subjekts,
- einen Lichtsensor (12) zum Empfangen mindestens eines Teils des ausgestrahlten Lichts nach einer Interaktion des ausgestrahlten Lichts mit dem Gewebe,
- eine Evaluierungseinheit (13) zum Ermitteln der elektrodermalen Aktivität aus dem empfangenen Licht, wobei die Evaluierungseinheit (12) konfiguriert ist, nur infrarotes Licht im Wellenlängenbereich zwischen 750 und 950 nm zu evaluieren, und
- eine Stütze (14) zum Tragen der Lichtquelle (11), des Lichtsensors (12) und der Evaluierungseinheit (13), wobei die Lichtquelle und der Lichtsensor bei einem vorbestimmten Abstand voneinander angeordnet sind.

2. Tragbare Vorrichtung nach Anspruch 1,
wobei die Lichtquelle (11) konfiguriert ist, nur infrarotes Licht im Wellenlängenbereich zwischen 750 nm und 950 nm auszustrahlen, insbesondere im Wellenlängenbereich zwischen 775 und 825 nm oder bei einer Wellenlänge von 800 nm.

3. Tragbare Vorrichtung nach Anspruch 1,
wobei der Lichtsensor (12) konfiguriert ist, nur infrarotes Licht im Wellenlängenbereich zwischen 750 und 950 nm zu empfangen, insbesondere im Wellenlängenbereich zwischen 775 und 825 nm oder bei einer Wellenlänge von 800 nm.

4. Tragbare Vorrichtung nach Anspruch 1,
wobei die Evaluierungseinheit (12) weiter konfiguriert ist, nur infrarotes Licht im Wellenlängenbereich zwischen 775 und 825 nm oder bei einer Wellenlänge von 800 nm zu evaluieren.

5. Tragbare Vorrichtung nach Anspruch 1,
wobei die Lichtquelle (11) und der Lichtsensor (12) bei einem Abstand voneinander im Bereich zwischen 0,5 mm und 5 mm angeordnet sind, insbesondere im Bereich zwischen 1 mm und 4 mm.

6. Tragbare Vorrichtung nach Anspruch 1,
wobei die Evaluierungseinheit (14) konfiguriert ist, die Intensität des empfangenen Lichts zu evaluieren, um die elektrodermale Aktivität zu ermitteln.

7. Tragbare Vorrichtung nach Anspruch 1,
weiter umfassend ein Halteelement (15) zum Halten der tragbaren Vorrichtung am Körper des Subjekts.

8. Tragbare Vorrichtung nach Anspruch 7,
wobei das Halteelement (15) ein Armband, einen Gürtel, einen Aufkleber, ein Band oder einen Klips umfasst.

9. Tragbare Vorrichtung nach Anspruch 1,
weiter umfassend eine Anwenderschnittstelle (16) zum Ausstellen von Informationen über die ermittelte elektrodermale Aktivität.

10. Verfahren zum Ermitteln elektrodermaler Aktivität eines Subjekts (1), das Verfahren umfassend:
- Ausstrahlen von Licht durch eine Lichtquelle (11), enthaltend infrarotes Licht im Wellenlängenbereich zwischen 750 und 950 nm, in Gewebe des Subjekts,
- Empfangen, durch einen Lichtsensor (12), mindestens eines Teils des ausgestrahlten Lichts nach einer Interaktion des ausgestrahlten Lichts mit dem Gewebe,
- Ermitteln, durch eine Evaluierungseinheit (13), der elektrodermalen Aktivität vom empfangenen Licht, wobei die Ermittlung auf Evaluierung von nur infrarotem Licht im Wellenlängenbereich zwischen 750 und 950 nm basiert,
- wobei die Lichtquelle (11), der Lichtsensor (12) und die Evaluierungseinheit (13) von einer Stütze (14) getragen werden und wobei die Lichtquelle und der Lichtsensor bei einem vorbestimmten Abstand voneinander angeordnet sind.

11. Verfahren nach Anspruch 10, weiter umfassend Evaluieren nur infraroten Lichts im Wellenlängenbereich zwischen 775 und 825 nm oder bei einer Wellenlänge von 800 nm.

12. Computerprogramm, umfassend Programmcodemittel zum Veranlassen einer Vorrichtung nach Anspruch 1, die Schritte des Verfahrens nach Anspruch 10 oder 11 durchzuführen.

## Revendications

1. Dispositif portable pour déterminer l'activité électrodermale d'un sujet (1), le dispositif portable (10) comprenant :
- une source de lumière (11) pour émettre de la lumière comprenant de la lumière infrarouge dans la plage de longueurs d'onde comprise entre 750 et 950 nm dans un tissu du sujet,
- un capteur de lumière (12) pour recevoir au moins une partie de la lumière émise après une interaction de la lumière émise avec le tissu,
- une unité d'évaluation (13) pour déterminer l'activité électrodermale de la lumière reçue, dans lequel ladite unité d'évaluation (12) est configurée pour évaluer uniquement la lumière infrarouge dans la plage de longueurs d'onde comprise entre 750 et 950 nm et
- un support (14) pour supporter la source de lumière (11), le capteur de lumière (12) et l'unité d'évaluation (13), dans lequel la source de lumière et le capteur de lumière sont agencés à une distance prédéterminée l'un de l'autre.

2. Dispositif portable selon la revendication 1,
dans lequel ladite source de lumière (11) est configurée pour émettre seulement de la lumière infrarouge dans la plage de longueurs d'onde comprise entre 750 et 950 nm, en particulier dans la plage de longueurs d'onde comprise entre 775 et 825 nm ou à une longueur d'onde de 800 nm.

3. Dispositif portable selon la revendication 1,
dans lequel ledit capteur de lumière (12) est configuré pour recevoir seulement de la lumière infrarouge dans la plage de longueurs d'onde comprise entre 750 et 950 nm, en particulier dans la plage de longueurs d'onde comprise entre 775 et 825 nm ou à une longueur d'onde de 800 nm.

4. Dispositif portable selon la revendication 1,
dans lequel ladite unité d'évaluation (12) est configurée en outre pour évaluer seulement de la lumière infrarouge dans la plage de longueurs d'onde comprise entre 750 et 825 nm, ou à une longueur d'onde de 800 nm.

5. Dispositif portable selon la revendication 1,
dans lequel la source de lumière (11) et le capteur de lumière (12) sont agencés à une distance dans la plage comprise entre 0,5 mm et 5 mm, en particulier dans la plage comprise entre 1 mm et 4 mm, l'un de l'autre.

6. Dispositif portable selon la revendication 1,
dans lequel ladite unité d'évaluation (14) est configurée pour évaluer l'intensité de la lumière reçue afin de déterminer l'activité électrodermale.

7. Dispositif portable selon la revendication 1,
comprenant en outre un élément de support (15) pour supporter le dispositif portable sur le corps du sujet.

8. Dispositif portable selon la revendication 7,
dans lequel ledit élément de support (15) comprend un bracelet, une ceinture, une autocollant, un ruban ou une pince.

9. Dispositif portable selon la revendication 1,
comprenant en outre une interface utilisateur (16) pour délivrer des informations sur l'activité électrodermale déterminée.

10. Procédé de détermination de l'activité électrodermale d'un sujet (1), ledit procédé comprenant :
- l'émission de lumière par une source de lumière (11) comprenant de la lumière infrarouge dans la plage de longueurs d'onde comprise entre 750 et 950 nm dans un tissu du sujet,
- la réception par un capteur de lumière (12) d'au moins une partie de la lumière émise après une interaction de la lumière émise avec le tissu et
- la détermination par une unité d'évaluation (13) de l'activité électrodermale de la lumière reçue, dans lequel la détermination est basée sur l'évaluation uniquement de la lumière infrarouge dans la plage de longueurs d'onde comprise entre 750 et 950 nm,
dans lequel la source de lumière (11), le capteur de lumière (12) et l'unité d'évaluation (13) sont portées par un support (14) et dans lequel la source de lumière et le capteur de lumière sont agencés à une distance prédéterminée l'un de l'autre.

11. Procédé selon la revendication 10, comprenant en outre l'évaluation seulement de la lumière infrarouge dans la plage de longueurs d'onde comprise entre 775 et 825 nm ou à une longueur d'onde de 800 nm.

12. Programme informatique comprenant des moyens de codage de programme pour amener un dispositif selon la revendication 1 à effectuer les étapes du procédé selon les revendications 10 ou 11.
